# EUROPEAN PATENT APPLICATION

(11) **EP 3 889 612 A1**
(43) Date of publication of application: **06.10.2021**
(21) Application number: 20166826.6
(22) Date of filing: 30.03.2020
(51) Int. Cl.: G01N 33/68

(54) **A METHOD OF DIAGNOSING AND/OR PROGNOSING PREECLAMPSIA**

(71) Applicant: University College Dublin, National University of Ireland, Dublin, Dublin 4 (IE)
(72) Inventor: MAGUIRE, Patricia, Dublin, 4 (IE); SZKLANNA, Pauline, Dublin, 4 (IE); NÍ ÁINLE, Fionnuala, Dublin, 4 (IE)
(74) Representative: FRKelly

(57) **Abstract**

The present invention relates to a method of diagnosing and/or prognosing preeclampsia. Specifically, the method involves determining the quantitative level of one or more biomarkers in a biological sample from the subject and either diagnosing preeclampsia; prognosing unstable moderate early-onset preeclampsia; and/or diagnosing preeclampsia and prognosing unstable moderate early-onset preeclampsia in the subject.

## Description

### Field of the Invention

The present invention relates to a method of diagnosing and/or prognosing preeclampsia in a subject. The method comprises determining the quantitative level of one or more biomarkers in a biological sample from the subject and either diagnosing preeclampsia, prognosing unstable moderate early-onset preeclampsia, and/or diagnosing preeclampsia and prognosing unstable moderate early-onset preeclampsia in the subject; based on the quantitative level of the or each biomarker in the biological sample.

### Background to the Invention

Preeclampsia (PE) is a disorder of pregnancy characterised by the onset of high blood pressure and often a significant amount of protein in the urine. When PE arises, the condition begins after 20 weeks of pregnancy. In severe disease there may be red blood cell breakdown, a low blood platelet count, impaired liver function, kidney dysfunction, swelling, shortness of breath due to fluid in the lungs, or visual disturbances. PE increases the risk of poor outcomes for both the mother and the baby. If left untreated, it may result in seizures at which point it is known as eclampsia. The cause of PE is still not fully understood, though the disease was recognised and described nearly 2000 years ago. At present, delivery of the pre-term baby is the only treatment for PE and the safest option for the mother.

PE affects 2-8% of pregnancies worldwide and severe cases develop in about 1 to 2% of pregnancies. PE is a leading cause of maternal and infant mortality with nearly 50,000 maternal and 500,000 infant deaths each year worldwide. Hypertensive disorders of pregnancy (which include preeclampsia) are one of the most common causes of death due to pregnancy. Women who have had preeclampsia are at increased risk of heart disease and stroke later in life; and infant prematurity carries increased risk of long- and short-term respiratory conditions, neurodevelopmental impairment and chronic health problems.

PE is diagnosed when a pregnant woman develops:
1. Blood pressure ≥140 mmHg systolic or ≥90 mmHg diastolic on two separate readings taken at least four to six hours apart after 20 weeks' gestation in an individual with previously normal blood pressure; or
2. In a woman with essential hypertension beginning before 20 weeks' gestational age, the diagnostic criteria are: an increase in systolic blood pressure (SBP) of ≥30 mmHg or an increase in diastolic blood pressure (DBP) of ≥15 mmHg; or
3. Proteinuria ≥ 0.3 grams (300 mg) or more of protein in a 24-hour urine sample or a SPOT urinary protein to creatinine ratio ≥0.3 or a urine dipstick reading of 1+ or greater (dipstick reading should only be used if other quantitative methods are not available).

PE is usually classified as mild or severe. In most settings, PE is classified as severe when any of the following conditions are present: severe hypertension, heavy proteinuria or substantial maternal organ dysfunction. PE usually occurs after 32 weeks; however, if it occurs earlier it is associated with worse outcomes. Early onset (before 32-34 weeks of pregnancy) preeclampsia (EOP) and foetal morbidity are used as independent criteria to classify PE as severe in some parts of the world. Maternal deaths can occur among severe cases, but the progression from mild to severe can be rapid, unexpected, and occasionally fulminant.

EOP can be further classified in terms of severity progression, as stable moderate early-onset preeclampsia (SM EOP) and unstable moderate early-onset preeclampsia (UM EOP). Severity of EOP drives a preterm delivery, associated with a significant risk of long-term neurodevelopmental infant morbidity and mortality; and accounts for a huge proportion of annual admissions to neonatal ICU. Even a minor improvement in gestation at delivery would confer a significant infant survival benefit, such that every hour in utero counts. Accurate risk stratification would reduce these enormous competing risks for mothers and babies, facilitating early intervention before severe complications have occurred. On the other hand, accurately identifying EOP which will not progress to a severe phenotype could prevent unnecessary preterm/emergency delivery with all its associated complications for both mothers and babies.

SM EOP is defined as moderate preeclampsia (according to NICE definition) at the time of admission and at delivery (severe preeclampsia according to the ACOG definition but excluding those with diastolic blood pressure (DBP) 90-99 mmHg and systolic blood pressure (SBP) 140-149 mmHg). UM EOP is defined as moderate preeclampsia at the time of admission (as described above), whose condition worsened and ultimately experienced severe preeclampsia (classified if blood pressure exceeds 160/110 mmHg with more pronounced proteinuria or when it is associated with thrombocytopenia (low platelet count), IUGR and/or liver damage) at the time of delivery (excluding those with DBP 90-99 mmHg and SBP 140-149 mmHg on admission).

Diagnosing PE and EOP is further complicated by the fact that signs and symptoms, such as headache and swollen ankles, are commonly seen in pregnancy and overlap with other medical conditions seen in pregnancy, such as liver disease, renal disease, chronic hypertension, and idiopathic thrombocytopenic purpura. Moreover, the standard clinical diagnostic criteria, such as hypertension or proteinuria, are not sufficiently accurate to predict adverse outcomes associated with the disease.

Thus, the problem/need is to diagnose PE, especially EOP, more effectively/specifically and to prognose SM EOP and UM EOP more effectively/specifically.

### Summary of the Invention

According to a first aspect of the present invention, there is provided a method of diagnosing preeclampsia in a subject, the method comprising the steps of:
(a) determining the quantitative level of one or more biomarkers in a biological sample from the subject; and
(b) diagnosing preeclampsia in the subject based on the quantitative level of the or each biomarker in the biological sample;
   wherein the biomarker is FN1.

Optionally or additionally, the method further comprises prognosing unstable moderate early-onset preeclampsia in the subject.

Optionally, there is provided a method of diagnosing preeclampsia, and/or prognosing unstable moderate early-onset preeclampsia in a subject, the method comprising the steps of:
(a) determining the quantitative level of one or more biomarkers in a biological sample from the subject; and
(b) diagnosing preeclampsia; and/or prognosing unstable moderate early-onset preeclampsia in the subject based on the quantitative level of the or each biomarker in the biological sample;
   wherein the biomarker is FN1.

According to a second aspect of the present invention, there is provided a method of diagnosing preeclampsia in a subject, the method comprising the steps of:
(a) determining the quantitative level of one or more biomarkers in a biological sample from the subject; and
(b) diagnosing preeclampsia in the subject based on the quantitative level of the or each biomarker in the biological sample;
   wherein the biomarker is CPB2.

Optionally or additionally, the method further comprises prognosing stable moderate early-onset preeclampsia in the subject.

Optionally, there is provided a method of diagnosing preeclampsia, and/or prognosing unstable moderate early-onset preeclampsia in a subject, the method comprising the steps of:
(a) determining the quantitative level of one or more biomarkers in a biological sample from the subject; and
(b) diagnosing preeclampsia; and/or prognosing unstable moderate early-onset preeclampsia in the subject based on the quantitative level of the or each biomarker in the biological sample;
   wherein the biomarker is CPB2.

Optionally, the or each biomarker for diagnosing preeclampsia, and/or prognosing unstable moderate early-onset preeclampsia comprises FN1 and CPB2.

Optionally, there is provided a method of diagnosing preeclampsia, and/or prognosing unstable moderate early-onset preeclampsia in a subject, the method comprising the steps of:
(a) determining the quantitative level of one or more biomarkers in a biological sample from the subject; and
(b) diagnosing preeclampsia; and/or prognosing unstable moderate early-onset preeclampsia in the subject based on the quantitative level of the or each biomarker in the biological sample;
   wherein the biomarkers are FN1 and CPB2.

Optionally, there is provided a method of diagnosing preeclampsia wherein the method of diagnosing preeclampsia is early-onset preeclampsia.

Further optionally, the or each biomarker for diagnosing preeclampsia, and/or prognosing unstable moderate early-onset preeclampsia further comprises ORM2, IGLC2, C5, C9, ENDOD1, FGA, HBD, PSG1, STOM, EHD1 and DNM1L.

Optionally, the or each biomarker for diagnosing preeclampsia, and/or prognosing unstable moderate early-onset preeclampsia comprises ORM2, IGLC2, C5, C9, CPB2, ENDOD1, FGA, HBD, PSG1, STOM, EHD1 and DNM1L.

Optionally, the or each biomarker for diagnosing preeclampsia, and/or prognosing unstable moderate early-onset preeclampsia comprises ORM2, IGLC2, C5, C9, ENDOD1, FGA, FN1, HBD, PSG1, STOM, EHD1 and DNM1L.

Optionally, the or each biomarker for diagnosing preeclampsia, and/or prognosing stable moderate early-onset preeclampsia is selected from ORM2, IGLC2, C5, C9, CPB2, ENDOD1, FGA, FN1, HBD, PSG1, STOM, EHD1 and DNM1L.

Optionally, the method is a method of diagnosing preeclampsia in a subject.

Optionally, the method is a method of diagnosing early-onset preeclampsia in a subject.

Optionally, there is provided a method of diagnosing preeclampsia in a subject, the method comprising the steps of:
(a) determining the quantitative level of one or more biomarkers in a biological sample from the subject; and
(b) diagnosing preeclampsia in the subject based on the quantitative level of the or each biomarker in the biological sample;
wherein the biomarker is FN1.

Optionally, the or each biomarker for diagnosing preeclampsia further comprises CPB2, ENDOD1 and EHD1.

Optionally, the or each biomarker for diagnosing preeclampsia comprises FN1, CPB2, ENDOD1 and EHD1.

Optionally, the or each biomarker for diagnosing preeclampsia further comprises C5, PSG1, DNM1L, PRDX2, IGLC2, ORM2, FBLN1, HBD, STOM, FGA and AMBP.

Optionally, the or each biomarker for diagnosing preeclampsia comprises FN1, CPB2, ENDOD1, EHD1, C5, PSG1, DNM1L, PRDX2, IGLC2, ORM2, FBLN1, HBD, STOM, FGA and AMBP .

Optionally, the or each biomarker for diagnosing preeclampsia is further comprises C3, GUSB, C9, CCT7, PSG2, CFB, HBB, SERPINA1, APOC3, FGG and IGHG2.

Optionally, the or each biomarker for diagnosing preeclampsia comprises FN1, CPB2, ENDOD1, EHD1, C5, PSG1, DNM1L, PRDX2, IGLC2, ORM2, FBLN1, HBD, STOM, FGA, AMBP, C3, GUSB, C9, CCT7, PSG2, CFB, HBB, SERPINA1, APOC3, FGG and IGHG2.

Optionally, the or each biomarker for diagnosing preeclampsia is selected from FN1, CPB2 ENDOD1, EHD1, C5, PSG1, DNM1L, PRDX2, IGLC2, ORM2, FBLN1, HBD, STOM, FGA, AMBP, GUSB, C9, CCT7, PSG2, CFB, HBB, SERPINA1, APOC3, FGG and IGHG2.

Optionally, the method of diagnosing preeclampsia includes early-onset preeclampsia.

Optionally, the method is a method of prognosing unstable moderate early-onset preeclampsia in a subject.

Optionally, there is provided a method of prognosing unstable moderate early-onset preeclampsia in a subject, the method comprising the steps of:
(a) determining the quantitative level of one or more biomarkers in a biological sample from the subject; and
(b) prognosing unstable moderate early-onset preeclampsia in the subject based on the quantitative level of the or each biomarker in the biological sample;
   wherein the or each biomarker is FN1.

Optionally, the or each biomarker for prognosing unstable moderate early-onset preeclampsia further comprises CPB2, C5, PSG1, DNM1L, IGLC2, HBD and ORM2.

Optionally, the or each biomarker for prognosing unstable moderate early-onset preeclampsia comprises FN1, CPB2, C5, PSG1, DNM1L, IGLC2, HBD and ORM2

Optionally, the or each biomarker for prognosing unstable moderate early-onset preeclampsia further comprises ENDOD1, EHD1, STOM, FGA, COL4A2, APOE, PSG9 and C9.

Optionally, the or each biomarker for prognosing unstable moderate early-onset preeclampsia comprises FN1, CPB2, C5, PSG1, DNM1L, IGLC2, HBD, ORM2 ENDOD1, EHD1, STOM, FGA, COL4A2, APOE, PSG9 and C9.

Optionally, the or each biomarker for prognosing unstable moderate early-onset preeclampsia further comprises ALB, PRKAR2B, LBP, HPSE, PSG3, SHBG, SVEP1, UBE2L3 and SERPIND1.

Optionally, the or each biomarker for prognosing unstable moderate early-onset preeclampsia comprises FN1, CPB2, C5, PSG1, DNM1L, IGLC2, HBD, ORM2 ENDOD1, EHD1, STOM, FGA, COL4A2, APOE, PSG9, C9, ALB, PRKAR2B, LBP, HPSE, PSG3, SHBG, SVEP1, UBE2L3 and SERPIND1.

Optionally, the or each biomarker for prognosing unstable moderate early-onset preeclampsia is selected from FN1, CPB2, C5, PSG1, DNM1L, PSG1, IGLC2, HBD, ORM2, ENDOD1, EHD1, STOM, FGA, COL4A2, APOE, PSG9, C9, ALB, PRKAR2B, LBP, HPSE, PSG3, SHBG, SVEP1, UBE2L3 and SERPIND1.

Optionally, the or each biomarker is a gene.

Optionally, the or each biomarker is a nucleic acid.

Optionally, the or each biomarker is a deoxyribonucleic acid.

Optionally, the or each biomarker is a ribonucleic acid.

Optionally, the or each biomarker is a protein.

Optionally, the or each biomarker is a peptide.

Optionally, the or each biomarker for diagnosing preeclampsia, and/or prognosing unstable moderate early-onset preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR and ESKPLTAQQTTK.

Preferably, the or each biomarker for diagnosing preeclampsia, and/or prognosing unstable moderate early-onset preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR and TYLGNALVCTCYGGSR for diagnosing preeclampsia.

Preferably, the or each biomarker for diagnosing preeclampsia, and/or prognosing unstable moderate early-onset preeclampsia is a peptide having an amino acid sequence ESKPLTAQQTTK for prognosing unstable moderate early-onset preeclampsia.

Preferably, the or each biomarker for diagnosing preeclampsia, and/or prognosing unstable moderate early-onset preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR for diagnosing preeclampsia; and ESKPLTAQQTTK for prognosing unstable moderate early-onset preeclampsia.

Further preferably, the or each biomarker for diagnosing preeclampsia, and/or prognosing unstable moderate early-onset preeclampsia is a peptide having an amino acid sequence IHIGSSFEK.

Optionally, the or each biomarker for diagnosing preeclampsia, and/or prognosing unstable moderate early-onset preeclampsia is a peptide having an amino acid sequence selected from any one or more FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR or IHIGSSFEK for diagnosing preeclampsia; and ESKPLTAQQTTK or IHIGSSFEK for prognosing unstable moderate early-onset preeclampsia.

Optionally, the or each biomarker for diagnosing preeclampsia, and/or prognosing unstable moderate early-onset preeclampsia is a peptide having an amino acid sequence selected from any one or more of NWGLSFYADKPETTK, EQLGEFYEALDCLCIPR, SYSCQVTHEGSTVEK, ATLVCLISDFYPGAVTVAWK, GGSASTWLTAFALR, MVETTAYALLTSLNLK, LSPIYNLVPVK, VVEESELAR, RPWNVASLIYETK, ALTPQCGSGEDLYILTGTVPSDYR, HPDEAAFFDTASTGK, VQHIQLLQK, GLIDEVNQDFTNR, VNVDAVGGEALGR, GTFSQLSELHCDK, FTFTLHLETPKPSISSSNLNPR, LPKPYITINNLNPR, EASMVITESPAALQLR, VIAAEGEMNASR, VYGALMWSLGK and/or IFSPNVVNLTLVDLPGMTK.

Further optionally, the or each biomarker for diagnosing preeclampsia, and/or prognosing unstable moderate early-onset preeclampsia is a peptide having an amino acid sequence selected from any one or more of NWGLSFYADKPETTK, EQLGEFYEALDCLCIPR, SYSCQVTHEGSTVEK, ATLVCLISDFYPGAVTVAWK, GGSASTWLTAFALR, MVETTAYALLTSLNLK, LSPIYNLVPVK, VVEESELAR, RPWNVASLIYETK, IHIGSSFEK, ALTPQCGSGEDLYILTGTVPSDYR, HPDEAAFFDTASTGK, VQHIQLLQK, GLIDEVNQDFTNR, FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, ESKPLTAQQTTK, VNVDAVGGEALGR, GTFSQLSELHCDK, FTFTLHLETPKPSISSSNLNPR, LPKPYITINNLNPR, EASMVITESPAALQLR, VIAAEGEMNASR, VYGALMWSLGK and/or IFSPNVVNLTLVDLPGMTK.

Preferably, the or each biomarker for diagnosing preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR or TYLGNALVCTCYGGSR.

Further preferably, the or each biomarker for diagnosing preeclampsia is a peptide having an amino acid sequence selected from any one or more of IHIGSSFEK, ALTPQCGSGEDLYILTGTVPSDYR and VYGALMWSLGK.

Optionally, the or each biomarker for diagnosing preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, IHIGSSFEK, ALTPQCGSGEDLYILTGTVPSDYR and VYGALMWSLGK.

Optionally, the or each biomarker for diagnosing preeclampsia is a peptide having an amino acid sequence further comprising GGSASTWLTAFALR, FTFTLHLETPKPSISSSNLNPR, IFSPNVVNLTLVDLPGMTK, LSEDYGVLK, EGGLGPLNIPLLADVTR, SYSCQVTHEGSTVEK, NWGLSFYADKPETTK, ATLVCLISDFYPGAVTVAWK, AITPPHPASQANIIFDITEGNLR, VNVDAVGGEALGR, EASMVITESPAALQLR, HPDEAAFFDTASTGK, ETLLQDFR and AFIQLWAFDAVK.

Optionally, the or each biomarker for diagnosing preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, IHIGSSFEK, ALTPQCGSGEDLYILTGTVPSDYR, VYGALMWSLGK, GGSASTWLTAFALR, FTFTLHLETPKPSISSSNLNPR, IFSPNVVNLTLVDLPGMTK, LSEDYGVLK, EGGLGPLNIPLLADVTR, SYSCQVTHEGSTVEK, NWGLSFYADKPETTK, ATLVCLISDFYPGAVTVAWK, AITPPHPASQANIIFDITEGNLR, VNVDAVGGEALGR, EASMVITESPAALQLR, HPDEAAFFDTASTGK, ETLLQDFR and AFIQLWAFDAVK.

Further optionally, the or each biomarker for diagnosing preeclampsia is a peptide having an amino acid sequence further comprising SSLSVPYVIVPLK, IPIEDGSGEVVLSR, SLLEQYHLGLDQK, LSPIYNLVPVK, SLHDAIMIVR, NSATGEESSTSLTVK, SDPVTLNLLHGPDLPR, DFHINLFQVLPWLK, GTFATLSELHCDK, LYHSEAFTVNFGDTEEAK, GWVTDGFSSLK, IHLISTQSAIPYALR and NQVSLTCLVK.

Still further optionally, the or each biomarker for diagnosing preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, IHIGSSFEK, ALTPQCGSGEDLYILTGTVPSDYR, VYGALMWSLGK, GGSASTWLTAFALR, FTFTLHLETPKPSISSSNLNPR, IFSPNVVNLTLVDLPGMTK, LSEDYGVLK, EGGLGPLNIPLLADVTR, SYSCQVTHEGSTVEK, NWGLSFYADKPETTK, ATLVCLISDFYPGAVTVAWK, AITPPHPASQANIIFDITEGNLR, VNVDAVGGEALGR, EASMVITESPAALQLR, HPDEAAFFDTASTGK, ETLLQDFR, IHIGSSFEK AFIQLWAFDAVK, SSLSVPYVIVPLK, IPIEDGSGEVVLSR, SLLEQYHLGLDQK, LSPIYNLVPVK, SLHDAIMIVR, NSATGEESSTSLTVK, SDPVTLNLLHGPDLPR, DFHINLFQVLPWLK, GTFATLSELHCDK, LYHSEAFTVNFGDTEEAK, GWVTDGFSSLK, IHLISTQSAIPYALR and NQVSLTCLVK.

Preferably, the biomarker for prognosing unstable moderate early-onset preeclampsia is a peptide having an amino acid sequence ESKPLTAQQTTK.

Further preferably, the or each biomarker for prognosing unstable moderate early-onset preeclampsia is a peptide having an amino acid sequence selected from any one or more of IHIGSSFEK, GGSASTWLTAFALR, MVETTAYALLTSLNLK, FTFTLHLETPKPSISSSNLNPR, LPKPYITINNLNPR, IFSPNVVNLTLVDLPGMTK, SYSCQVTHEGSTVEK, GTFSQLSELHCDK and EQLGEFYEALDCLCIPR.

Optionally, the or each biomarker for prognosing unstable moderate early-onset preeclampsia is a peptide having an amino acid sequence selected from any one or more of ESKPLTAQQTTK, IHIGSSFEK, GGSASTWLTAFALR, MVETTAYALLTSLNLK, FTFTLHLETPKPSISSSNLNPR, LPKPYITINNLNPR, IFSPNVVNLTLVDLPGMTK, SYSCQVTHEGSTVEK, GTFSQLSELHCDK and EQLGEFYEALDCLCIPR.

Further optionally, the or each biomarker for prognosing unstable moderate early-onset preeclampsia is a peptide having an amino acid sequence further comprising ALTPQCGSGEDLYILTGTVPSDYR, VYGALMWSLGK, VIAAEGEMNASR, VQHIQLLQK, SVSIGYLLVK, VQAAVGTSAAPVPSDNH, SNPVILNVLYGPDLPR, GLIDEVNQDFTNR, VVEESELAR and RPWNVASLIYETK.

Further optionally, the or each biomarker for prognosing unstable moderate early-onset preeclampsia is a peptide having an amino acid sequence selected from any one or more of IHIGSSFEK, GGSASTWLTAFALR, MVETTAYALLTSLNLK, FTFTLHLETPKPSISSSNLNPR, LPKPYITINNLNPR, IFSPNVVNLTLVDLPGMTK, SYSCQVTHEGSTVEK, GTFSQLSELHCDK, EQLGEFYEALDCLCIPR, ALTPQCGSGEDLYILTGTVPSDYR, VYGALMWSLGK, VIAAEGEMNASR, VQHIQLLQK, SVSIGYLLVK, VQAAVGTSAAPVPSDNH, SNPVILNVLYGPDLPR, GLIDEVNQDFTNR, VVEESELAR and RPWNVASLIYETK.

Still further optionally, the or each biomarker for prognosing unstable moderate early-onset preeclampsia is a peptide having an amino acid sequence selected further comprising DVFLGMFLYEYAR, LVRPEVDVMCTAFHDNEETFLK, AATITATSPGALWGLDR, ITLPDFTGDLR, TDFLIFDPK, LFIPQITTK, DIPQPHAEPWAFSLDLGLK, LLSDFPVVPTATR, IEINFPAEYPFKPPK and FPVEMTHNHNFR.

Still further optionally, the or each biomarker for prognosing unstable moderate early-onset preeclampsia is a peptide having an amino acid sequence selected from any one or more of ESKPLTAQQTTK, IHIGSSFEK, GGSASTWLTAFALR, MVETTAYALLTSLNLK, FTFTLHLETPKPSISSSNLNPR, IFSPNVVNLTLVDLPGMTK, LPKPYITINNLNPR, SYSCQVTHEGSTVEK, GTFSQLSELHCDK, EQLGEFYEALDCLCIPR, ALTPQCGSGEDLYILTGTVPSDYR, VYGALMWSLGK, VIAAEGEMNASR, VQHIQLLQK, SVSIGYLLVK, VQAAVGTSAAPVPSDNH, SNPVILNVLYGPDLPR, GLIDEVNQDFTNR, VVEESELAR, RPWNVASLIYETK, DVFLGMFLYEYAR, LVRPEVDVMCTAFHDNEETFLK, AATITATSPGALWGLDR, ITLPDFTGDLR, TDFLIFDPK, LFIPQITTK, DIPQPHAEPWAFSLDLGLK, LLSDFPVVPTATR, IEINFPAEYPFKPPK and FPVEMTHNHNFR.

Still further optionally, the or each biomarker for prognosing unstable moderate early-onset preeclampsia is selected from VQAAVGTSAAPVPSDNH, GGSASTWLTAFALR, MVETTAYALLTSLNLK, SVSIGYLLVK, IHIGSSFEK, IFSPNVVNLTLVDLPGMTK, ITLPDFTGDLR, AATITATSPGALWGLDR, FTFTLHLETPKPSISSSNLNPR, LPKPYITINNLNPR, LFIPQITTK and LLSDFPVVPTATR.

Optionally, the determining step (a) comprises determining the quantitative level of one or more biomarkers in the biological sample from the subject.

Further optionally, the determining step (a) comprises determining the quantitative level of two, three, four, five, six, seven, eight, nine, ten, eleven, twelve, thirteen, fourteen, fifteen, twenty, twenty five, thirty or more biomarkers in the biological sample from the subject.

Optionally, the determining step (a) comprises determining the quantitative level of all of the biomarkers in the biological sample from the subject.

Optionally or additionally, the determining step (a) comprises determining the quantitative level of each of the biomarkers in the biological sample from the subject.

Optionally, the diagnosing and/or prognosing step (b) comprises comparing the quantitative level of the or each biomarker in the biological sample from the subject with the quantitative level of the or each respective biomarkers in a normal sample.

Optionally, in the method of diagnosing preeclampsia, and/or prognosing unstable moderate early-onset preeclampsia, the biological sample is selected from whole blood, serum, plasma, urine, interstitial fluid, peritoneal fluid, cervical swab, tears, saliva, buccal swab, skin, brain tissue, and cerebrospinal fluid.

Further optionally, in the method of diagnosing preeclampsia, and/or prognosing unstable moderate early-onset preeclampsia, the normal sample is a biological sample from a subject not suffering from preeclampsia.

Still further optionally, in the method of diagnosing preeclampsia, and/or prognosing unstable moderate early-onset preeclampsia, the normal sample is a biological sample from a healthy pregnant subject.

Optionally, in the method of diagnosing preeclampsia, the biological sample is selected from whole blood, serum, plasma, urine, interstitial fluid, peritoneal fluid, cervical swab, tears, saliva, buccal swab, skin, brain tissue, and cerebrospinal fluid.

Further optionally, in the method of diagnosing preeclampsia, the normal sample is a biological sample from a subject not suffering from preeclampsia.

Still further optionally, in the method of diagnosing preeclampsia, the normal sample is a biological sample from a healthy pregnant subject.

Optionally, in the method of prognosing unstable moderate early-onset preeclampsia, the biological sample is selected from whole blood, serum, plasma, urine, interstitial fluid, peritoneal fluid, cervical swab, tears, saliva, buccal swab, skin, brain tissue, and cerebrospinal fluid.

Further optionally, in the method of prognosing unstable moderate early-onset preeclampsia, the normal sample is a biological sample from a subject not suffering from preeclampsia.

Still further optionally, in the method of prognosing unstable moderate early-onset preeclampsia, the normal sample is a biological sample from a healthy pregnant subject.

Optionally, the determining step (a) further comprises determining the quantitative level in a first set of respective biomarkers(s).

Optionally, the determining step (a) further comprises determining the quantitative level a first set of respective biomarkers(s) wherein the quantitative level of the first set of biomarkers is greater than the quantitative level the respective biomarkers(s) in a normal sample is indicative of preeclampsia.

Optionally, the first set of biomarkers is selected from FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, VYGALMWSLGK, GGSASTWLTAFALR, LSEDYGVLK, EGGLGPLNIPLLADVTR, NWGLSFYADKPETTK, AITPPHPASQANIIFDITEGNLR, VNVDAVGGEALGR, EASMVITESPAALQLR, HPDEAAFFDTASTGK, ETLLQDFR, AFIQLWAFDAVK, SSLSVPYVIVPLK, SLHDAIMIVR, NSATGEESSTSLTVK, GTFATLSELHCDK, LYHSEAFTVNFGDTEEAK, GWVTDGFSSLK and IHLISTQSAIPYALR.

Further optionally, the first set of biomarkers is selected from FN1, EHD1, C5, PRDX2, ORM2, FBLN1, HBD, STOM, FGA, AMBP, C3, CCT7, PSG2, HBB, SERPINA1, APOC3 and FGG.

Alternatively, the determining step (a) further comprises determining the quantitative level in a second set of respective biomarkers(s).

Alternatively, the determining step (a) further comprises determining the quantitative level a second set of respective biomarkers(s) wherein the quantitative level of the second set of biomarkers is less than the quantitative level the respective biomarkers(s) in a normal sample is indicative of preeclampsia.

Optionally, the second set of biomarkers is selected from IHIGSSFEK, ALTPQCGSGEDLYILTGTVPSDYR, FTFTLHLETPKPSISSSNLNPR, IFSPNVVNLTLVDLPGMTK, SYSCQVTHEGSTVEK, ATLVCLISDFYPGAVTVAWK, IPIEDGSGEVVLSR, SLLEQYHLGLDQK, LSPIYNLVPVK, SDPVTLNLLHGPDLPR, DFHINLFQVLPWLK and NQVSLTCLVK.

Further optionally, the second set of biomarkers is selected from CPB2, ENDOD1, PSG1, DNM1L, IGLC2, C3, GUSB, C9, PSG2, CFB and IGHG2.

Further optionally, the determining step (a) further comprises determining the quantitative level in a third set of respective biomarkers(s).

Further optionally, the determining step (a) further comprises determining the quantitative level a third set of respective biomarkers(s) wherein the quantitative level of the third set of biomarkers is greater than the quantitative level the respective biomarkers(s) in a normal sample is indicative of unstable moderate early-onset preeclampsia.

Optionally, the third set of biomarkers is selected from GGSASTWLTAFALR, MVETTAYALLTSLNLK, FTFTLHLETPKPSISSSNLNPR, LPKPYITINNLNPR, SYSCQVTHEGSTVEK, EQLGEFYEALDCLCIPR, VYGALMWSLGK, SVSIGYLLVK, VQAAVGTSAAPVPSDNH, SNPVILNVLYGPDLPR, RPWNVASLIYETK, DVFLGMFLYEYAR, LVRPEVDVMCTAFHDNEETFLK, AATITATSPGALWGLDR, ITLPDFTGDLR and FPVEMTHNHNFR.

Further optionally, the third set of biomarkers is selected from C5, PSG1, IGLC2, ORM2, EHD1, COL4A2, APOE, PSG9, C9, ALB, PRKAR2B, LBP and SERPIND1.

Further alternatively, the determining step (a) further comprises determining the quantitative level in a fourth set of respective biomarkers(s).

Further alternatively, the determining step (a) further comprises determining the quantitative level a fourth set of respective biomarkers(s) wherein the quantitative level of the fourth set of biomarkers is less than the quantitative level the respective biomarkers(s) in a normal sample is indicative of unstable moderate early-onset preeclampsia.

Optionally, the fourth set of biomarkers is selected from ESKPLTAQQTTK, IHIGSSFEK, IFSPNVVNLTLVDLPGMTK, GTFSQLSELHCDK, ALTPQCGSGEDLYILTGTVPSDYR, VIAAEGEMNASR, VQHIQLLQK, GLIDEVNQDFTNR, VVEESELAR, TDFLIFDPK, LFIPQITTK, DIPQPHAEPWAFSLDLGLK, LLSDFPVVPTATR and IEINFPAEYPFKPPK.

Further optionally, the fourth set of biomarkers is selected from FN1, CBP2, DNM1L, HBD, ENDOD1, STOM, FGA, C9, HPSE, PSG3, SHBG, SVEP1 and UBE2L3.

Optionally, the method of diagnosing preeclampsia is early-onset preeclampsia.

Optionally, the method of prognosing unstable moderate early-onset preeclampsia further comprises prognosing stable moderate early-onset preeclampsia.

Optionally, the method of prognosing unstable moderate early-onset preeclampsia further comprises differentiating unstable moderate early-onset preeclampsia from stable moderate early-onset preeclampsia.

Optionally, the method of diagnosing preeclampsia, and/or prognosing unstable moderate early-onset preeclampsia is an in vitro method.

Optionally, the method of diagnosing preeclampsia, and/or prognosing unstable moderate preeclampsia is an in vitro method wherein the preeclampsia is early-onset preeclampsia.

Optionally, the method of diagnosing preeclampsia is an in vitro method.

Optionally, the method of diagnosing early-onset preeclampsia is an in vitro method.

Optionally, the method of prognosing unstable moderate early-onset preeclampsia is an in vitro method.

According to a further aspect of the present invention, there is provided a method of treating preeclampsia, and/or unstable moderate early-onset preeclampsia by diagnosing preeclampsia; and/or prognosing unstable moderate early-onset preeclampsia; and treating the subject. Optionally, the method of treating preeclampsia, and/or unstable moderate early-onset preeclampsia by diagnosing preeclampsia; and/or prognosing unstable moderate early-onset preeclampsia includes early-onset preeclampsia; and treating the subject.

### Brief Description of the Drawings

Embodiments of the present invention will now be described with reference to the accompanying drawings and following non-limiting examples, in which:
**Figure 1** illustrates mass spectrometry analysis of pregnancy and early-onset preeclampsia (EOP) platelet releasate;
**Figure 2** illustrates that 36 peptides are differentially expressed in EOP patients;
**Figure 3** illustrates that 36 differentially expressed peptides are capable of 100% separation of patients into healthy pregnancy or EOP group;
**Figure 4** illustrates that 30 peptides are differentially expressed in UM EOP patients; and
**Figure 5** illustrates that 30 differentially expressed peptides are capable of 100% separation of patients into SM EOP or UM EOP.

### Materials & Methods

### Patient recruitment

Following an informed consent, patients were recruited from the Rotunda Hospital in Dublin. Early-onset preeclampsia (EOP) was diagnosed before 34 weeks of gestation with a new-onset hypertension above 140/90 mmHg measured on two separate occasions, proteinuria ≥ 0.3 g/24 hrs or 1 + on a dipstick test, low platelet count, IUGR presence and the evidence of abnormal blood flow. Patients were classified as severe EOP if blood pressure exceeded 160/110 mmHg with proteinuria ≥ 1.5 g/24 hrs (or 2+ on a dipstick test). Patients were classified into 3 groups based on their disease progression:
1. Patients with moderate preeclampsia at the time of admission and at delivery but excluding those with diastolic blood pressure (DBP) 90-99 mmHg and systolic blood pressure (SBP) 140-149 mmHg); termed stable moderate early onset preeclampsia (SM EOP);
2. Patients with severe preeclampsia at the time of admission and delivery; termed stable severe preeclampsia (SS EOP); and
3. Patients with moderate preeclampsia at the time of admission, whose condition worsened and ultimately experienced severe PE at the time of delivery (excluding those with DBP 90-99 mmHg and SBP 140-149 mmHg on admission); termed unstable moderate preeclampsia (UM EOP).

### Platelet releasate isolation

Human platelets were obtained from 18 healthy pregnant and 22 early-onset preeclampsia patients in accordance with approved guidelines from University College Dublin and the Rotunda Hospital, Dublin. Human platelet releasate was isolated from washed platelets. Blood was drawn from human volunteers free from medication into 0.15% v/v acid/citrate/dextrose (ACD) anticoagulant (38 mM anhydrous citric acid, 75 mM sodium citrate, 124 mM D-glucose). Blood was centrifuged at 150g for 10 minutes at room temperature and platelet rich plasma (PRP) aspirated. Platelets were pelleted from PRP by centrifugation at 720g for 10 min at room temperature and resuspended in a modified Tyrodes buffer (JNL) (130 mM NaCl, 10 mM trisodium citrate, 9 mM NaHCO₃, 6 mM dextrose, 0.9 mM MgCl₂, 0.81 mM KH₂PO₄, 10 mM Tris pH 7.4) and supplemented with 1.8 mM CaCl₂. Platelet counts were adjusted to 2 x 10⁸ / mL using a Sysmex™ haematology analyser (TOA Medical Electronics, Kobe, Japan). Platelets were activated with 1 U/ml thrombin (Roche, Basel, Switzerland) under constant stirring (1000 rpm) for 5 mins using a Chronolog-700 platelet aggregometer (Chronolog Cor, Manchester, UK). Platelet aggregates were removed by centrifugation by centrifugation three times at 10,000 xg for 10 minutes. Harvested platelet releasates were stored at - 80 °C until subsequent use.

### Sample preparation and mass spectrometry

PR samples were solubilised in RIPA buffer and proteins precipitated overnight with 95% acetone (4:1 acetone: sample volume) at -20 °C. Dried protein pellets were resuspended in 8M urea/24mM Tris-HCL, pH 8.2, at 37 °C for one hour. Disulphide bonds were reduced with 5mM DTT and protected with 15mM iodoacetamide. PR samples were digested with Lys-C (1:100; Promega, Madison, WI) followed by digestion with trypsin (1:100; Promega). Peptides were purified using ZipTipC₁₈ pipette tips (Millipore, Billerica, MA, USA) and resuspended in 1% formic acid. For data-dependent acquisition (DDA), samples were analysed using a Thermo-Scientific Q-Exactive mass spectrometer connected to a Dionex Ultimate 3000 (RSLCnano) liquid chromatography (LC) system. In brief, each 5 µg sample was loaded onto a fused silica emitter (75 µm ID), pulled using a laser puller (Sutter Instruments P2000, Novato, CA, USA), packed with Reprocil Pur (Dr Maisch, Ammerbuch-Entringen, Germany) C18 (1.9 µm; 12 cm in length) reverse-phase media and separated by an increasing acetonitrile gradient over 47 minutes (flow rate = 250 nL/min) direct into a Q-Exactive MS. The MS was operated in positive ion mode with a capillary temperature of 320 °C, and with a potential of 2300 V applied to the frit. All data was acquired while operating in automatic data-dependent switching mode. A high resolution (70,000) MS scan (300-1600 m/z) was performed using the Q Exactive to select the 12 most intense ions prior to MS/MS analysis using high-energy collision dissociation (HCD).

The data-independent acquisition (DIA) isolation scheme and multiplexing strategy was based on that from Egertson et al. (2013) in which five 4-*m*/*z* isolation windows are analysed per scan. DIA data was acquired for the psychotic experiences group (Table 1; 40Cases/66 Controls). Samples were run on the Thermo Scientific Q Exactive mass spectrometer in DIA mode. Each DIA cycle contained one full MS-SIM scan and 20 DIA scans covering a mass range of 490-910^{Th} with the following settings: the SIM full scan resolution was 35,000; AGC 1e6; Max IT 55ms; profile mode; DIA scans were set at a resolution of 17,000; AGC target 1e5; Max IT 20ms; loop count 10; MSX count 5; 4.0 m/z isolation windows; centroid mode (Egertson et al. 2013). The cycle time was 2s, which resulted in at least ten scans across the precursor peak. For the library, QC samples were injected in DDA mode at the beginning of the run, and after every ten injections throughout the run. The relative fragment-ion intensities, peptide-precursor isotope peaks and retention time of the extracted ion chromatograms from the DIA files were used to confirm the identity of the target molecular species.

### Protein identification and quantification

Raw DDA MS files were analysed by MaxQuant (MQ) version 1.5.0.30. MS/MS spectra were searched by the Andromeda search engine against a human FASTA (August 2016) obtained from UniProt. MQ analysis included an initial search with a precursor mass tolerance of 20 ppm the results of which were used for mass recalibration. In the main Andromeda search precursor mass and fragment mass had an initial mass tolerance of 6 ppm and 20 ppm, respectively. The search included fixed modification of carbamidomethyl cysteine. Minimal peptide length was set to 7 amino acids and a maximum of 2 miscleavages was allowed. The false discovery rate (FDR) was set to 0.01 for peptide/protein identification. For quantitative comparison between samples we used label-free quantification (LFQ) with a minimum of two ratio counts to determine the normalized protein intensity. LFQ intensities were assigned to identified proteins by comparing the area under the curve of the signal intensity for any given peptide. The total protein approach (TPA) was used for quantitative comparison, to determine protein abundance as a fraction of total protein.

All DIA data was processed in the open-source Skyline software tool (open-source Skyline software tool (https://skyline.gs.washington.edu). This tool provided the interface for visual confirmation of protein biomarkers in the samples profiled, without any file conversion. The library was constructed from previously described MaxQuant analysis. As detailed in the online tutorials and publications by the Skyline team, the msms.txt file resulting from the MaxQuant search was used to build the library in Skyline. For our peptide targets, mass chromatograms were extracted for +2 and +3 precursor charge states and their associated fragment ions. Based on our discovery results, we analysed 89 protein candidates. For our dataset, the *m*/*z* tolerance was <10ppm and the average retention time window was 2 minutes. All parent and fragment level data was visually confirmed across the samples run, and peak editing was undertaken where necessary, using the peptide Retention Time (RT), dotproduct (idop), mass accuracy (<10ppm), and a confirmed library match to reliably identify and quantify peptides across the DIA runs. For statistical analysis, peak areas of the fragment level data was filtered from the Skyline document grid for analysis in mapDIA, an open source bioinformatics tool for pre-processing and quantitative analysis of DIA data. Retention time normalisation procedure was applied, followed by peptide fragment selection using 2 standard deviation threshold for outlier detection, in the independent sample setup. Differential expression analysis was analysed in Perseus software.

Data was processed in the Perseus open framework (http://www.perseus-framework.org). Protein IDs were filtered to eliminate identifications from the reverse database, proteins only identified by site, and common contaminants. TPA values were Log₂ transformed. A protein was included if it was identified in at least 50% of samples in at least one group. Random forest analysis with either 80:20% or 60:40% data split was performed using Waikato Environment for Knowledge Analysis (WEKA).

### Results

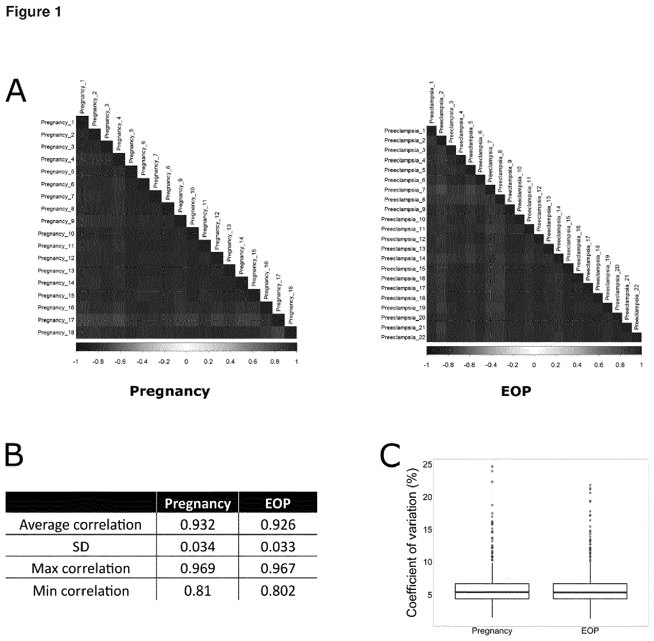

Pearson correlation coefficient (r) and coefficient of variation (CV) analysis was performed to assess biological variability in protein abundances. We found strong inter-donor reproducibility across our PR samples, averaging at r = 0.932 ± 0.034 for healthy pregnant controls (Figure 1 (A&B)) and r = 0.926 ± 0.033 for EOP patients (including stable moderate (SM), unstable moderate (UM) and stable severe (SS) EOP patients) (Figure 1 (A&B)). The average variance in protein quantitation was very low both in pregnant control (average CV = 5.8% ± 2.5%) and EOP PR (average CV = 5.9% ± 2.6%), indicating that PR contents were highly reproducible across donors. Moreover, maximum variance was low in both pregnant control and EOP PR (24.6% and 21.9% respectively; Figure 1 (C)). Collectively, these data suggest that the PR is a stable proteome in healthy pregnant and EOP women with vast majority of proteins exhibiting < 20% associated variability.

### Example 1 - mass spectrometry analysis of pregnancy and early-onset preeclampsia (EOP) platelet releasate exhibits very good correlation.

Referring to Figure 1(A), strong correlations (> 0.8) were found in the 18 healthy pregnancies and 22 EOP platelet releasate (PR) samples. Figure 1(B) shows a table listing average, maximum and minimum correlations as well as standard deviation for 18 healthy pregnancy and 22 EOP PR samples. Figure 1(C) illustrates pregnancy and EOP platelet releasate proteomes show little variation with an average coefficient of variation (CV) 5.8% ± 2.5% and 5.9% ± 2.6% respectively. The maximum variance was lower for the EOP PR (CV = 21.9%) when compared to pregnancy PR (CV = 24.6%).

From our discovery (DDA) mass spectrometry analysis we have uncovered 89 protein candidates to analyse through DIA. For 9 of 89 proteins we were not able to identify MS1 or MS2 spectra through our DIA methodology and further 2 did not pass DIA inclusion criteria, therefore these were removed from further analysis. Table 1 contains the complete list (78) of the proteins analysed.

**Table 1. A list of proteins analysed through Skyline software following data-independent acquisition method.**

| **Gene name** | **Protein name** | **UniProt ID** |
|---|---|---|
| FN1 | Fibronectin | P02751 |
| C5 | Complement C5 | P01031 |
| PSG1 | Pregnancy-specific beta-1-glycoprotein 1 | P11464 |
| PRDX2 | Peroxiredoxin-2 | P32119 |
| DNM1L | Dynamin-1-like protein | 000429 |
| ENDOD1 | Endonuclease domain-containing 1 protein | 094919 |
| CFHR1 | Complement factor H-related protein 1 | Q03591 |
| ORM2 | Alpha-1-acid glycoprotein 2 | P19652 |
| IGLC2 | Immunoglobulin lambda constant 2 | P0DOY2 |
| FBLN1 | Fibulin-1 | P23142 |
| COL4A2 | Collagen alpha-2 | P08572 |
| HBD | Hemoglobin subunit delta | P02042 |
| APOE | Apolipoprotein E | P02649 |
| STOM | Erythrocyte band 7 integral membrane protein | P27105 |
| AMBP | Protein AMBP [Cleaved into: Alpha-1-microglobulin | P02760 |
| PSG9 | Pregnancy-specific beta-1-glycoprotein 9 | Q00887 |
| EHD1 | EH domain-containing protein 1 | Q9H4M9 |
| FGA | Fibrinogen alpha chain [Cleaved into: Fibrinopeptide A; Fibrinogen alpha chain] | P02671 |
| C3 | Complement C3 | P01024 |
| C7 | Complement component C7 | P10643 |
| CPB2 | Carboxypeptidase B2 | Q96IY4 |
| GUSB | Beta-glucuronidase | P08236 |
| CCT7 | T-complex protein 1 subunit eta | Q99832 |
| IGHV5-10-1 | Immunoglobulin heavy variable 5-10-1 | A0A0J9YXX1 |
| PSG2 | Pregnancy-specific beta-1-glycoprotein 2 | P11465 |
| CFB | cDNA FLJ55673, highly similar to Complement factor B | B4E1Z4 |
| ALB | Serum albumin | P02768 |
| HBB | Hemoglobin subunit beta | P68871 |
| PRKAR2B | cAMP-dependent protein kinase type II-beta | P31323 |
| | regulatory subunit | |
| LBP | Lipopolysaccharide-binding protein | P18428 |
| C9 | Complement component C9 [Cleaved into: Complement component C9a; Complement component C9b] | P02748 |
| HBA1 | Hemoglobin subunit alpha | P69905 |
| SERPINA1 | Alpha-1-antitrypsin | P01009 |
| HPSE | Heparanase | Q9Y251 |
| PSG3 | Pregnancy-specific beta-1-glycoprotein 3 | Q16557 |
| TGFBI | Transforming growth factor-beta-induced protein ig-h3 | Q15582 |
| SHBG | Sex hormone-binding globulin | P04278 |
| IGHV3-33 | Immunoglobulin heavy variable 3-33 | P01772 |
| SVEP1 | Sushi, von Willebrand factor type A, EGF and pentraxin domain-containing protein 1 | Q4LDE5 |
| CP | Ceruloplasmin | P00450 |
| CLEC3B | Tetranectin | P05452 |
| UBE2L3 | Ubiquitin-conjugating enzyme E2 L3 | P68036 |
| SERPIND1 | Heparin cofactor 2 | P05546 |
| APOC3 | Apolipoprotein C-III | P02656 |
| NUTF2 | Nuclear transport factor 2 | P61970 |
| CTSC | Dipeptidyl peptidase 1 | P53634 |
| AGT | Angiotensinogen | P01019 |
| AHSG | Alpha-2-HS-glycoprotein | P02765 |
| FGG | Fibrinogen gamma chain | P02679 |
| PRG2 | Bone marrow proteoglycan | P13727 |
| PDCD6IP | Programmed cell death 6-interacting protein | Q8WUM4 |
| IGHG2 | Immunoglobulin heavy constant gamma 2 | P01859 |
| MMRN1 | Multimerin-1 | Q13201 |
| ST6GAL1 | Beta-galactoside alpha-2,6-sialyltransferase 1 | P15907 |
| VTN | Vitronectin | P04004 |
| APOC2 | Apolipoprotein C-II | P02655 |
| C1S | Complement C1s subcomponent | P09871 |
| CAND1 | Cullin-associated NEDD8-dissociated protein 1 | Q86VP6 |
| CD84 | SLAM family member 5 | Q9UIB8 |
| CSH2 | Chorionic somatomammotropin hormone 2 | P0DML3 |
| DMTN | Dematin | Q08495 |
| EEF1A1P5 | Putative elongation factor 1-alpha-like 3 | Q5VTE0 |
| F12 | Coagulation factor XII | P00748 |
| FETUB | Fetuin-B | Q9UGM5 |
| GANAB | Neutral alpha-glucosidase AB | Q14697 |
| HINT1 | Histidine triad nucleotide-binding protein 1 | P49773 |
| HRG | Histidine-rich glycoprotein | P04196 |
| IGKV2-24 | Immunoglobulin kappa variable 2-24 | AOAOC4DH68 |
| ITIH3 | Inter-alpha-trypsin inhibitor heavy chain H3 | Q06033 |
| KNG1 | Kininogen-1 | P01042 |
| MMP1 | Interstitial collagenase | P03956 |
| ORM1 | Alpha-1-acid glycoprotein 1 | P02763 |
| PF4 | Platelet factor 4 | P02776 |
| PLG | Plasminogen | P00747 |
| PSG7 | Putative pregnancy-specific beta-1-glycoprotein 7 | Q13046 |
| PZP | Pregnancy zone protein | P20742 |
| SERPINA7 | Thyroxine-binding globulin | P05543 |
| UGP2 | UTP--glucose-1-phosphate uridylyltransferase | Q16851 |

Skyline software allows for an analysis of individual peptides identified for each of the target proteins. Utilising mapDIA software, the area under the peak for each peptide fragments was used to assign quantitative value to a corresponding peptide. Furthermore, the quantitative data was normalised to retention time. Missing values were handled by imputation of a constant number equal to the lowest value x 0.9 for any given peptide. The quantitative peptide data was subjected to statistical analysis in Perseus software.

### Example 2 - Peptide expression in EOP patients

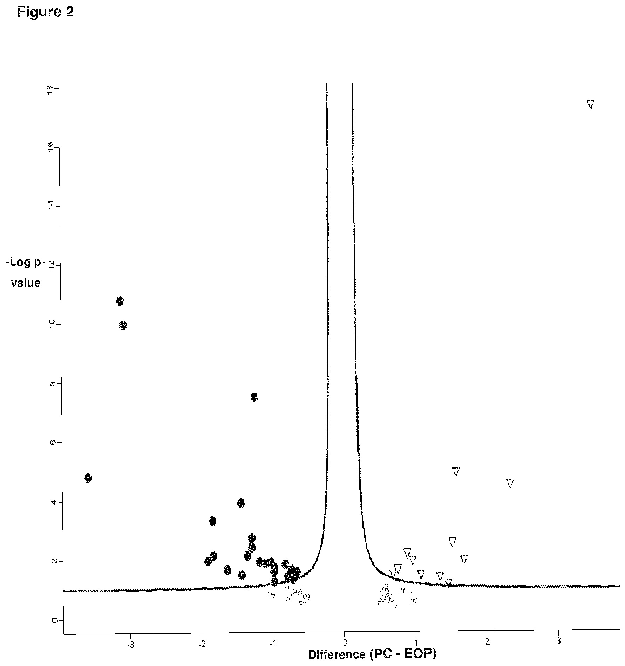

A peptide level analysis was carried out to assess the diagnosis of EOP further. 207 peptides from 60 proteins were quantified by mapDIA and were subjected to statistical analysis. Student's t-test analysis with an FDR of 5% and S₀ of 0.1 (denoted by the black hyperbolic lines, Figure 2) was performed on the 71 peptides meeting the minimal difference criteria (> 0.5 /< -0.5). 36 peptides were found differentially expressed in EOP samples, with 12 peptides decreased (right; IHIGSSFEK, ALTPQCGSGEDLYILTGTVPSDYR, FTFTLHLETPKPSISSSNLNPR, IFSPNVVNLTLVDLPGMTK, SYSCQVTHEGSTVEK, ATLVCLISDFYPGAVTVAWK, IPIEDGSGEVVLSR, SLLEQYHLGLDQK, LSPIYNLVPVK, SDPVTLNLLHGPDLPR, DFHINLFQVLPWLK, NQVSLTCLVK) and 24 peptides increased (left; FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, VYGALMWSLGK, GGSASTWLTAFALR, LSEDYGVLK, EGGLGPLNIPLLADVTR, NWGLSFYADKPETTK, AITPPHPASQANIIFDITEGNLR, VNVDAVGGEALGR, EASMVITESPAALQLR, HPDEAAFFDTASTGK, ETLLQDFR, AFIQLWAFDAVK, SSLSVPYVIVPLK, SLHDAIMIVR, NSATGEESSTSLTVK, GTFATLSELHCDK, LYHSEAFTVNFGDTEEAK, GWVTDGFSSLK, IHLISTQSAIPYALR) in EOP.

Figure 2 shows a volcano plot of healthy pregnancy vs EOP PR proteomes representing the peptides significantly altered in EOP patients. The black hyperbolic curved lines show the threshold for statistical significance, with an FDR of 0.05 and a minimal difference of 0.1. Our analysis revealed that the secretion levels of 24 peptides were significantly increased in EOP PR (circle) whereas 12 peptides were significantly decreased in EOP PR (triangle).

### Example 3 - Peptide separation of healthy pregnant and EOP patients

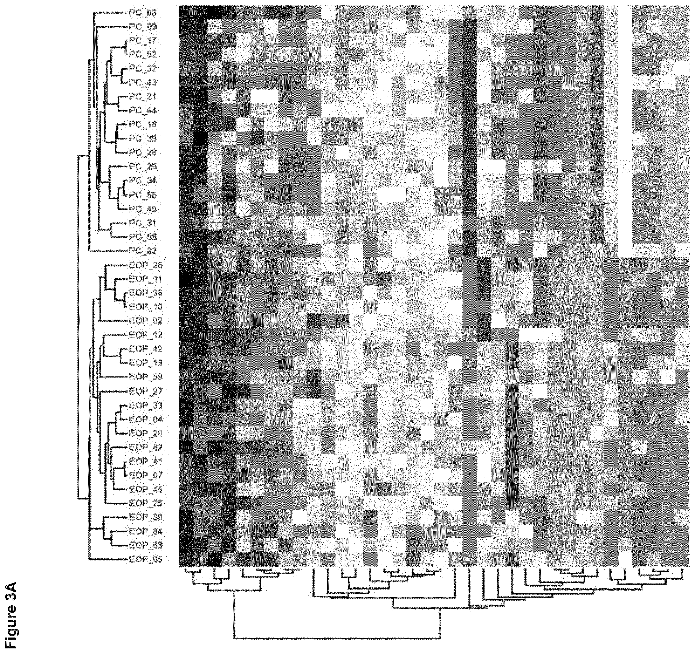

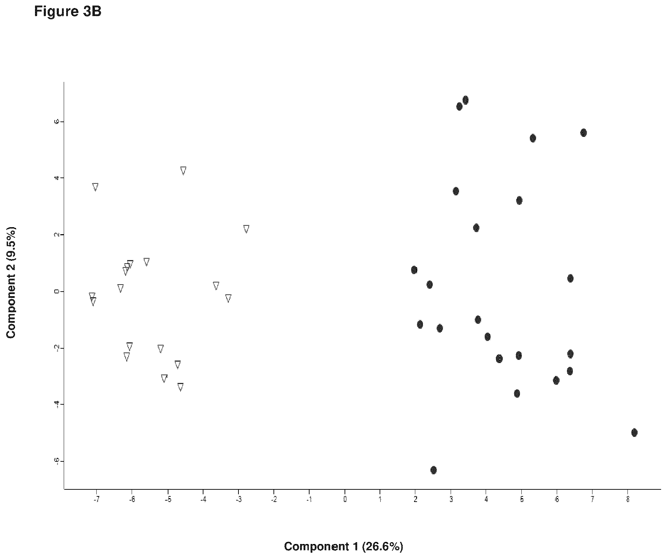

The hierarchical clustering and principal component analysis of the 36 differential proteins illustrate a complete separation of healthy pregnant and EOP patients (Figure 3).

Figure 3(A) illustrates euclidian distance based two-way hierarchical clustering of the log² transformed quantitative values of the 36 peptides (24 increased and 12 decreased) differentially released in EOP PR completely separates the samples into pregnancy and EOP clusters. Figure 3(B) illustrates principal component analysis of log² transformed quantitative values of the 36 differential peptides. Combination of component 1 and 2 enables complete separation of healthy pregnancy (triangle) and EOP (circle) PR samples.

The peptides deemed differentially expressed in EOP samples were furthermore subjected to machine learning analysis with the WEKA software. The peptides were subjected to InfoGain attribute selection and ranked according to their ability to separate the groups (Table 2).

**Table 2. Attribute selection ranking for the 36 differential EOP peptides.**

| **Rank** | **Gene names** | **Peptide** | **SEQ ID** |
|---|---|---|---|
| 1 | FN1 | FGFCPMAAHEEICTTNEGVMYR | SEQ ID NO.1 |
| 1 | FN1 | YSFCTDHTVLVQTR | SEQ ID NO.2 |
| 1 | FN1 | VDVIPVNLPGEHGQR | SEQ ID NO.3 |
| 1 | FN1 | EATIPGHLNSYTIK | SEQ ID NO.4 |
| 1 | FN1 | IYLYTLNDNAR | SEQ ID NO.5 |
| 1 | FN1 | TYLGNALVCTCYGGSR | SEQ ID NO.6 |
| 7 | CPB2 | IHIGSSFEK | SEQ ID NO.7 |
| 8 | ENDOD1 | ALTPQCGSGEDLYILTGTVPSDYR | SEQ ID NO.8 |
| 9 | EHD1 | VYGALMWSLGK | SEQ ID NO.9 |
| 10 | C5 | GGSASTWLTAFALR | SEQ ID NO.10 |
| 11 | PSG1 | FTFTLHLETPKPSISSSNLNPR | SEQ ID NO.11 |
| 12 | DNM1L | IFSPNVVNLTLVDLPGMTK | SEQ ID NO.12 |
| 13 | PRDX2 | LSEDYGVLK | SEQ ID NO.13 |
| 14 | PRDX2 | EGGLGPLNIPLLADVTR | SEQ ID NO.14 |
| 15 | IGLC2 | SYSCQVTHEGSTVEK | SEQ ID NO.15 |
| 16 | ORM2 | NWGLSFYADKPETTK | SEQ ID NO.16 |
| 17 | IGLC2 | ATLVCLISDFYPGAVTVAWK | SEQ 10 NO.17 |
| 18 | FBLN1 | AITPPHPASQANIIFDITEGNLR | SEQ 10 NO.18 |
| 19 | HBD | VNVDAVGGEALGR | SEQ 10 NO.19 |
| 20 | STOM | EASMVITESPAALQLR | SEQ ID NO.20 |
| 21 | FGA | HPDEAAFFDTASTGK | SEQ ID NO.21 |
| 22 | AMBP | ETLLQDFR | SEQ ID NO.22 |
| 23 | AMBP | IHIGSSFEK | SEQ ID NO.23 |
| 24 | C3 | SSLSVPYVIVPLK | SEQ ID NO.24 |
| 25 | C3 | IPIEDGSGEVVLSR | SEQ ID NO.25 |
| 26 | GUSB | SLLEQYHLGLDQK | SEQ ID NO.26 |
| 27 | C9 | LSPIYNLVPVK | SEQ ID NO.27 |
| 28 | CCT7 | SLHDAIMIVR | SEQ ID NO.28 |
| 29 | PSG2 | NSATGEESSTSLTVK | SEQ ID NO.28 |
| 30 | PSG2 | SDPVTLNLLHGPDLPR | SEQ ID NO.30 |
| 31 | CFB | DFHINLFQVLPWLK | SEQ ID NO.31 |
| 32 | HBB | GTFATLSELHCDK | SEQ ID NO.32 |
| 33 | SERPINA1 | LYHSEAFTVNFGDTEEAK | SEQ ID NO.33 |
| 34 | APOC3 | GWVTDGFSSLK | SEQ ID NO.34 |
| 35 | FGG | IHLISTQSAIPYALR | SEQ ID NO.35 |
| 36 | IGHG2 | NQVSLTCLVK | SEQ ID NO.36 |

The top 9 peptides (Table 3) were then used in a Random forest analysis where 80% of the data (EOP n=18, PC n=14) was used for training and 20% of the data (EOP n=4, PC n=4) was used for validation resulted in ROC AUC = 1, Specificity = 100% and Sensitivity = 100%. The addition of further peptides did not improve classification.

**Table 3. Random forest classification of the healthy pregnant and EOP patients based on the 9 top peptides.**

| Classified as: | Healthy pregnant | EOP |
|---|---|---|
| Healthy pregnant | **4** | 0 |
| EOP | 0 | **4** |

### Example 4 - Peptide expression in UM EOP patients

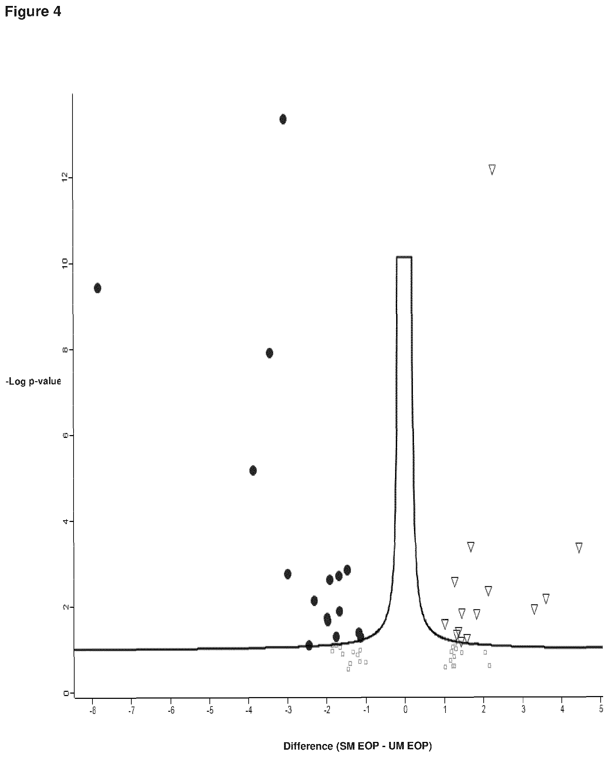

A peptide level analysis was carried out to assess the risk stratification for UM EOP further. 204 peptides from 63 proteins were quantified by mapDIA and were subjected to statistical analysis. Student's t-test analysis with an FDR of 5% and S0 of 0.1 (denoted by the black hyperbolic lines, (Figure 4) was performed on the 54 peptides meeting the minimal difference criteria (> 1 /< -1). 30 peptides were found differentially expressed in UM EOP samples, with 14 peptides decreased (right; ESKPLTAQQTTK, IHIGSSFEK, IFSPNVVNLTLVDLPGMTK, GTFSQLSELHCDK, ALTPQCGSGEDLYILTGTVPSDYR, VIAAEGEMNASR, VQHIQLLQK, GLIDEVNQDFTNR, VVEESELAR, TDFLIFDPK, LFIPQITTK, DIPQPHAEPWAFSLDLGLK, LLSDFPVVPTATR, IEINFPAEYPFKPPK) and 16 peptides increased (left; GGSASTWLTAFALR, MVETTAYALLTSLNLK, FTFTLHLETPKPSISSSNLNPR, LPKPYITINNLNPR, SYSCQVTHEGSTVEK, EQLGEFYEALDCLCIPR, VYGALMWSLGK, SVSIGYLLVK, VQAAVGTSAAPVPSDNH, SNPVILNVLYGPDLPR, RPWNVASLIYETK, DVFLGMFLYEYAR, LVRPEVDVMCTAFHDNEETFLK, AATITATSPGALWGLDR, ITLPDFTGDLR, FPVEMTHNHNFR) in UM EOP compared to SM EOP.

Figure 4 illustrates a volcano plot representing the peptides significantly altered in UM EOP patients. The black hyperbolic curved lines show the threshold for statistical significance, with an FDR of 0.05 and a minimal difference of 0.1. Our analysis revealed that the secretion levels of 16 peptides were significantly increased in UM EOP PR (circle) whereas 14 peptides were significantly decreased in UM EOP PR (triangle).

### Example 5 - Peptide separation of patients into SM EOP or UM EOP

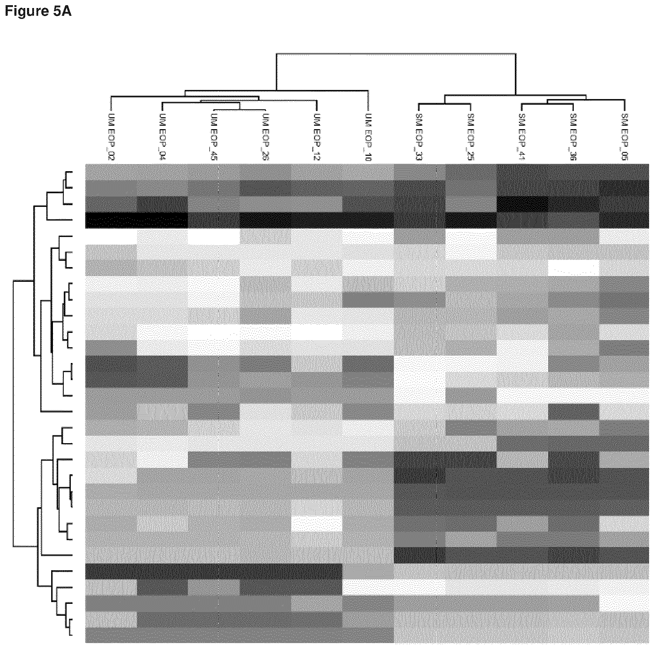

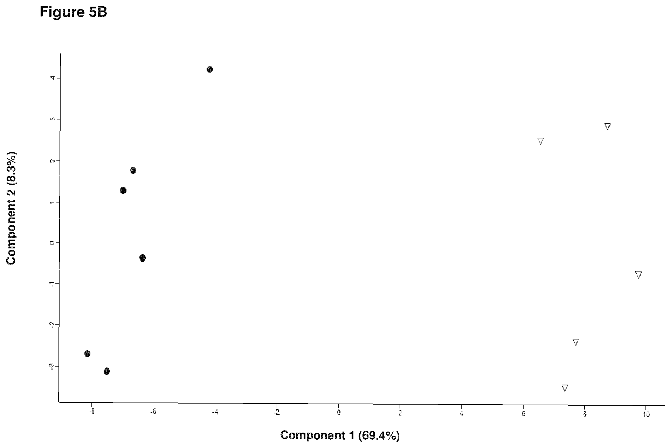

The hierarchical clustering and principal component analysis of the 30 differential peptides illustrate a complete separation of SM EOP and UM EOP patients (Figure 5).

Figure 5(A) illustrates euclidian distance based two-way hierarchical clustering of the log² transformed quantitative values of the 30 peptides (16 increased and 14 decreased) differentially released in UM EOP PR completely separates the samples into SM EOP and UM EOP clusters.

Figure 5(B) illustrates principal component analysis of log² transformed quantitative values of the 30 differential peptides. Combination of component 1 and 2 enables complete separation of SM EOP (triangle) and UM EOP (circle) PR samples.

The peptides deemed differentially expressed in SM and UM EOP samples were furthermore subjected to machine learning analysis with the WEKA software. The peptides were subjected to InfoGain attribute selection and ranked according to their ability to separate the groups (Table 4).

**Table 4. Attribute selection ranking for the 30 differential UM EOP peptides.**

| **Rank** | **Gene name** | **Peptide** | **SEQ ID** |
|---|---|---|---|
| 1 | FN1 | ESKPLTAQQTTK | SEQ ID NO.37 |
| 2 | CPB2 | IHIGSSFEK | SEQ ID NO.7 |
| 3 | C5 | GGSASTWLTAFALR | SEQ ID NO.10 |
| 4 | C5 | MVETTAYALLTSLNLK | SEQ ID NO.38 |
| 5 | PSG1 | FTFTLHLETPKPSISSSNLNPR | SEQ ID NO.11 |
| 6 | DNM1L | IFSPNVVNLTLVDLPGMTK | SEQ 10 NO.12 |
| 7 | PSG1 | LPKPYITINNLNPR | SEQ ID NO.39 |
| 8 | IGLC2 | SYSCQVTHEGSTVEK | SEQ ID NO.15 |
| 9 | HBD | GTFSQLSELHCDK | SEQ ID NO.40 |
| 10 | ORM2 | EQLGEFYEALDCLCIPR | SEQ ID NO.41 |
| 11 | ENDOD1 | ALTPQCGSGEDLYILTGTVPSDYR | SEQ ID NO.8 |
| 12 | EHD1 | VYGALMWSLGK | SEQ ID NO.9 |
| 13 | STOM | VIAAEGEMNASR | SEQ ID NO.42 |
| 14 | FGA | VQHIQLLQK | SEQ ID NO.43 |
| 15 | COL4A2 | SVSIGYLLVK | SEQ ID NO.44 |
| 16 | APOE | VQAAVGTSAAPVPSDNH | SEQ ID NO.45 |
| 17 | PSG9 | SNPVILNVLYGPDLPR | SEQ ID NO.46 |
| 18 | FGA | GLIDEVNQDFTNR | SEQ ID NO.47 |
| 19 | C9 | VVEESELAR | SEQ ID NO.48 |
| 20 | C9 | RPWNVASLIYETK | SEQ ID NO.49 |
| 21 | ALB | DVFLGMFLYEYAR | SEQ ID NO.50 |
| 22 | ALB | LVRPEVDVMCTAFHDNEETFLK | SEQ ID NO.51 |
| 23 | PRKAR2B | AATITATSPGALWGLDR | SEQ ID NO.52 |
| 24 | LBP | ITLPDFTGDLR | SEQ ID NO.53 |
| 25 | HPSE | TDFLIFDPK | SEQ ID NO.54 |
| 26 | PSG3 | LFIPQITTK | SEQ ID NO.55 |
| 27 | SHBG | DIPQPHAEPWAFSLDLGLK | SEQ ID NO.56 |
| 28 | SVEP1 | LLSDFPVVPTATR | SEQ ID NO.57 |
| 29 | UBE2L3 | IEINFPAEYPFKPPK | SEQ ID NO.58 |
| 30 | SERPIND1 | FPVEMTHNHNFR | SEQ ID NO.59 |

The top 10 peptides (Table 5) were then used in a Random forest analysis where 60% of the data (SM EOP n=2, UM EOP n=5) was used for training and 40% of the data (SM EOP n=3, UM EOP n=1) was used for validation resulted in ROC AUC = 1, Specificity = 100% and Sensitivity = 100%. The addition of further peptides did not improve classification.

**Table 5. Random forest classification of the SM EOP and UM EOP patients based on the 11 top peptides.**

| Classified as: | SM EOP | UM EOP |
|---|---|---|
| SM EOP | **3** | 0 |
| UM EOP | 0 | **1** |

**Table 6. - Overlapping proteins and peptides that diagnose preeclampsia; and/or prognose unstable moderate early-onset preeclampsia.**

| **Gene Name** | **Peptide in diagnostic panel** | **Peptide in prognostic panel** | **Protein** | **PE v Normal** | **UM EOP v SM EOP** |
|---|---|---|---|---|---|
| **ORM2** | NWGLSFYADKPETTK (SEQ ID.16) | EQLGEFYEAL DCLCIPR (SEQ ID.41) | Alpha-1-acid glycoprotei n2 | a b <-- classified as | a b <-- classified as |
| | | | | 3 1 \| a = PC | 3 1 \| a = PC |
| | | | | 0 4 \| b = PE | 0 4 \| b = PE |
| | | | | | |
| **IGLC2** | SYSCQVTHEGSTVEK (SEQ ID.15); | SYSCQVTHEG STVEK (SEQ ID.15) | Immunoglo bulin lambda constant 2 | a b <-- classified as | a b <-- classified as |
| | | | | 3 1 \| a : PC | 3 1 \| a = PC |
| | ATLVCLISDFYPGAVTV AWK (SEQ ID.17) | | | 1 3 \| b = PE | 0 4 \| b = PE |
| **C5** | GGSASTWLTAFALR (SEQ ID.10) | GGSASTWLTA FALR (SEQ ID.10); | Compleme nt C5 | a b <-- classified as 3 1 \| a = PC | a b <-- classified as 3 1 \| a = PC |
| | | MVETTAYALLT SLNLK (SEQ ID.38) | | 0 4 \| b = PE | 0 4 \| b = PE |
| **C9** | LSPIYNLVPVK (SEQ ID.27) | VVEESELAR (SEQ ID.48); | Compleme nt component C9 | a b <-- classified as 1 3 \| a = PC | a b <-- classified as 3 1 \| a = PC |
| | | RPWNVASLIY ETK (SEQ ID.49) | | 1 3 \| b = PE | 1 3 \| b = PE |
| **CPB2** | IHIGSSFEK (SEQ ID.7) | IHIGSSFEK (SEQ ID.7) | Carboxype ptidase B2 | a b <-- classified as | a b <-- classified as |
| | | | | 4 0 \| a = PC | 3 1 \| a = PC |
| | | | | 0 4 \| b = PE | 0 4 \| b = PE |
| **ENDO D1** | ALTPQCGSGEDLYILTG TVPSDYR (SEQ ID.8) | ALTPQCGSGE DLYILTGTVPS DYR (SEQ ID.8) | Endonucle ase domain-containing 1 protein | a b <-- classified as | a b <-- classified as |
| | | | | 4 0 \| a = PC | 3 1 \| a = PC |
| | | | | 0 4 \| b = PE | 0 4 \| b = PE |
| **FGA** | HPDEAAFFDTASTGK (SEQ ID.21) | VQHIQLLQK (SEQ ID.43); | Fibrinogen alpha chain | a b <-- classified as | a b <-- classified as |
| | | | | 2 2 \| a = PC | 1 3 \| a = PC |
| | | GLIDEVNQDFT NR (SEQ ID.47) | | 0 4 \| b = PE | \| 3 \| b = PE |
| **FN1** | FGFCPMAAHEEICTTN EGVMYR (SEQ ID.1); YSFCTDHTVLVQTR (SEQ ID.2); | ESKPLTAQQT TK (SEQ ID.37) | Fibronectin | a b <-- classified as | a b <-- classified as |
| | | | | 4 0 \| a = PC | 3 1 \| a = PC |
| | | | | 0 4 \| b = PE | 0 4 \| b = PE |
| | VDVIPVNLPGEHGQR (SEQ ID.3); | | | | |
| | EATIPGHLNSYTIK (SEQ ID.4); | | | | |
| | IYLYTLNDNAR (SEQ ID.5); | | | | |
| | TYLGNAL VCTCYGGSR (SEQ ID.6) | | | | |
| **HBD** | VNVDAVGGEALGR (SEQ ID.19) | GTFSQLSELH CDK (SEQ ID.40) | Hemoglobi n subunit delta | a b <-- classified as | a b <-- classified as |
| | | | | 1 3 \| a = PC | 2 1 \| a = PEMN |
| | | | | 0 4 \| b = PE | 0 1 \| b = PEMS |
| **PSG1** | FTFTLHLETPKPSISSS NLNPR (SEQ ID.11) | FTFTLHLETPK PSISSSNLNPR (SEQ ID.11); | Pregnancy-specific beta-1-glycoprotei n 1 | a b <-- classified as | a b <-- classified as |
| | | | | 3 1 \| a = PC | 3 1 \| a = PC |
| | | LPKPYITINNLN PR (SEQ ID.39) | | 2 2 \| b = PE | 0 4 \| b = PE |
| **STOM** | EASMVITESPAALQLR (SEQ ID.20) | VIAAEGEMNA SR (SEQ ID.42) | Erythrocyte band 7 integral membrane protein | a b <-- classified as | a b <-- classified as |
| | | | | 2 2 \| a = PC | 4 0 \| a = PC |
| | | | | 1 3 \| b = PE | 0 4 \| b = PE |
| **EHD1** | VYGALMWSLGK (SEQ ID.9) | VYGALMWSLG K (SEQ ID.9) | EH domain-containing protein 1 | a b <-- classified as | a b <-- classified as |
| | | | | 4 0 \| a = PC | 2 1 \| a = PEMM |
| | | | | 0 4 \| b = PE | 1 0 I b = PEMS |
| **DNM1 L** | IFSPNVVNLTLVDLPGM TK (SEQ ID.12) | IFSPNVVNLTL VDLPGMTK (SEQ ID.12) | Dynamin 1 Like protein | a b <-- classified as | a b <-- classified as |
| | | | | 3 1 \| a = PC | 3 0 \| a = PEMM |
| | | | | 0 4 \| b = PE | 0 1 \| b = PEMS |

Table 6 illustrates the proteins and peptides that diagnose preeclampsia, and/or prognose unstable moderate early-onset preeclampsia.

CPB2, ENDOD1 and FN1 diagnose preeclampsia with 100% specificity.

ORM2, IGLC2, C5, CBP2, FN1, HBD and PSG1 progonse UM EOP with 100% specificity.

## Claims

1. A method of diagnosing preeclampsia, and/or prognosing unstable moderate early-onset preeclampsia, in a subject; the method comprising the steps of:
(a) determining the quantitative level of one or more biomarkers in a biological sample from the subject; and
(b) diagnosing preeclampsia, and/or prognosing unstable moderate early-onset preeclampsia in the subject based on the quantitative level of the or each biomarker in the biological sample; wherein the or each biomarker is selected from FN1, CPB2, ORM2, IGLC2, C5, C9, ENDOD1, FGA, HBD, PSG1, STOM, EHD1 and DNM1L.

2. The method according to claim 1 wherein the method is a method of diagnosing preeclampsia, and the or each biomarker is selected from FN1, CPB2, ENDOD1 and EHD1.

3. The method according to claim 2 wherein the or each biomarker further comprises C5, PSG1, DNM1L, PRDX2, IGLC2, ORM2, FBLN1, HBD, STOM, FGA, AMBP, C3, GUSB, C9, CCT7, PSG2, CFB, HBB, SERPINA1, APOC3, FGG and IGHG2.

4. The method according to claim 1 wherein the method is a method of prognosing unstable moderate early-onset preeclampsia, and the or each biomarker is selected from FN1, CPB2, C5, PSG1, DNM1L, IGLC2, HBD and ORM2.

5. The method according to claim 4 wherein the or each biomarker further comprises ENDOD1, EHD1, STOM, FGA, COL4A2, APOE, PSG9, C9, ALB, PRKAR2B, LBP, HPSE, PSG3, SHBG, SVEP1, UBE2L3 and SERPIND1.

6. The method according to claim 1 wherein the or each biomarker for diagnosing preeclampsia, and/or prognosing unstable moderate early-onset preeclampsia is a peptide having an amino acid sequence selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, ESKPLTAQQTTK, IHIGSSFEK, NWGLSFYADKPETTK, EQLGEFYEALDCLCIPR, SYSCQVTHEGSTVEK, ATLVCLISDFYPGAVTVAWK, GGSASTWLTAFALR, MVETTAYALLTSLNLK, LSPIYNLVPVK, VVEESELAR, RPWNVASLIYETK, ALTPQCGSGEDLYILTGTVPSDYR, HPDEAAFFDTASTGK, VQHIQLLQK, GLIDEVNQDFTNR, VNVDAVGGEALGR, GTFSQLSELHCDK, FTFTLHLETPKPSISSSNLNPR, LPKPYITINNLNPR, EASMVITESPAALQLR, VIAAEGEMNASR, VYGALMWSLGK and IFSPNVVNLTLVDLPGMTK .

7. The method according to claim 2 wherein the or each biomarker is a peptide having an amino acid sequence selected from any one or more of FGFCPMAAHEEICTTNEGVMYR, YSFCTDHTVLVQTR, VDVIPVNLPGEHGQR, EATIPGHLNSYTIK,IYLYTLNDNAR, TYLGNALVCTCYGGSR, IHIGSSFEK, ALTPQCGSGEDLYILTGTVPSDYR and VYGALMWSLGK.

8. The method according to claim 3 wherein the or each biomarker is a peptide having an amino acid sequence selected from any one or more of GGSASTWLTAFALR, FTFTLHLETPKPSISSSNLNPR, IFSPNVVNLTLVDLPGMTK, LSEDYGVLK, EGGLGPLNIPLLADVTR, SYSCQVTHEGSTVEK, NWGLSFYADKPETTK, ATLVCLISDFYPGAVTVAWK, AITPPHPASQANIIFDITEGNLR, VNVDAVGGEALGR, EASMVITESPAALQLR, HPDEAAFFDTASTGK, ETLLQDFR, AFIQLWAFDAVK, SSLSVPYVIVPLK, IPIEDGSGEVVLSR, SLLEQYHLGLDQK, LSPIYNLVPVK, SLHDAIMIVR, NSATGEESSTSLTVK, SDPVTLNLLHGPDLPR, DFHINLFQVLPWLK, GTFATLSELHCDK, LYHSEAFTVNFGDTEEAK, GWVTDGFSSLK, IHLISTQSAIPYALR and NQVSLTCLVK.

9. The method according to claim 4 wherein the biomarker is a peptide having an amino acid sequence selected from any one or more of ESKPLTAQQTTK, IHIGSSFEK, GGSASTWLTAFALR, MVETTAYALLTSLNLK, FTFTLHLETPKPSISSSNLNPR, LPKPYITINNLNPR, IFSPNVVNLTLVDLPGMTK, SYSCQVTHEGSTVEK, GTFSQLSELHCDK and EQLGEFYEALDCLCIPR.

10. The method according to claim 5 wherein the or each biomarker is a peptide having an amino acid sequence selected from any one or more of ALTPQCGSGEDLYILTGTVPSDYR, VYGALMWSLGK, VIAAEGEMNASR, VQHIQLLQK, SVSIGYLLVK, VQAAVGTSAAPVPSDNH, SNPVILNVLYGPDLPR, GLIDEVNQDFTNR, VVEESELAR, RPWNVASLIYETK, DVFLGMFLYEYAR, LVRPEVDVMCTAFHDNEETFLK, AATITATSPGALWGLDR, ITLPDFTGDLR, TDFLIFDPK, LFIPQITTK, DIPQPHAEPWAFSLDLGLK, LLSDFPVVPTATR, IEINFPAEYPFKPPK and FPVEMTHNHNFR.

11. The method according to claim 8 wherein the determining step (a) further comprises determining the quantitative level in a first set of respective biomarkers(s), wherein the quantitative level of the first set of biomarkers is greater than the quantitative level the respective biomarkers(s) in a normal sample is indicative of preeclampsia, the first set of biomarkers is selected from any one or more of FGFCPMAAHEEICTTNEGVMYR, YSFCTDHTVLVQTR, VDVIPVNLPGEHGQR, EATIPGHLNSYTIK, IYLYTLNDNAR, TYLGNALVCTCYGGSR, VYGALMWSLGK, GGSASTWLTAFALR, LSEDYGVLK, EGGLGPLNIPLLADVTR, NWGLSFYADKPETTK, AITPPHPASQANIIFDITEGNLR, VNVDAVGGEALGR, EASMVITESPAALQLR, HPDEAAFFDTASTGK, ETLLQDFR, AFIQLWAFDAVK, SSLSVPYVIVPLK, SLHDAIMIVR, NSATGEESSTSLTVK, GTFATLSELHCDK, LYHSEAFTVNFGDTEEAK, GWVTDGFSSLK and IHLISTQSAIPYALR.

12. The method according to claim 8 wherein the determining step (a) further comprises determining the quantitative level in a second set of respective biomarkers(s), wherein the quantitative level of the second set of respective biomarkers(s) is less than the quantitative level the respective biomarkers(s) in a normal sample is indicative of preeclampsia, the second set of biomarkers is selected from any one or more of IHIGSSFEK, ALTPQCGSGEDLYILTGTVPSDYR, FTFTLHLETPKPSISSSNLNPR, IFSPNVVNLTLVDLPGMTK, SYSCQVTHEGSTVEK, ATLVCLISDFYPGAVTVAWK, IPIEDGSGEVVLSR, SLLEQYHLGLDQK, LSPIYNLVPVK, SDPVTLNLLHGPDLPR, DFHINLFQVLPWLK and NQVSLTCLVK.

13. The method according to claim 9 wherein the determining step (a) further comprises determining the quantitative level in a third set of respective biomarkers(s), wherein the quantitative level of the third set of respective biomarkers(s) is greater than the quantitative level the respective biomarkers(s) in a normal sample is indicative of unstable moderate early-onset preeclampsia, the third set of biomarkers is selected from any one or more of GGSASTWLTAFALR, MVETTAYALLTSLNLK, FTFTLHLETPKPSISSSNLNPR, LPKPYITINNLNPR, SYSCQVTHEGSTVEK, EQLGEFYEALDCLCIPR, VYGALMWSLGK, SVSIGYLLVK, VQAAVGTSAAPVPSDNH, SNPVILNVLYGPDLPR, RPWNVASLIYETK, DVFLGMFLYEYAR, LVRPEVDVMCTAFHDNEETFLK, AATITATSPGALWGLDR, ITLPDFTGDLR and FPVEMTHNHNFR.

14. The method according to claim 9 wherein the determining step (a) further comprises determining the quantitative level in a fourth set of respective biomarkers(s), wherein the quantitative level of the fourth set of respective biomarkers(s) is less than the quantitative level the respective biomarkers(s) in a normal sample is indicative of unstable moderate early-onset preeclampsia, the fourth set of biomarkers is selected from any one or more of ESKPLTAQQTTK, IHIGSSFEK, IFSPNVVNLTLVDLPGMTK, GTFSQLSELHCDK, ALTPQCGSGEDLYILTGTVPSDYR, VIAAEGEMNASR, VQHIQLLQK, GLIDEVNQDFTNR, VVEESELAR, TDFLIFDPK, LFIPQITTK, DIPQPHAEPWAFSLDLGLK, LLSDFPVVPTATR and IEINFPAEYPFKPPK.

15. The method according to any one of claims 1-3, 6-8 and 11-12 wherein the method of diagnosing preeclampsia is a method of diagnosing early-onset preeclampsia in a subject.


SEQUENCE LISTING

<110>  University College Dublin, National University of Ireland,
       Dublin

<120>  A method of diagnosing and/or prognosing preeclampsia

<130>  P132043EP00

<160>  59

<170>  PatentIn version 3.5

<210>  1
<211>  22
<212>  PRT
<213>  Unknown

<220>
<223>  Synthetic

<400>  1

Phe Gly Phe Cys Pro Met Ala Ala His Glu Glu Ile Cys Thr Thr Asn
1               5                   10                  15

Glu Gly Val Met Tyr Arg
            20

<210>  2
<211>  14
<212>  PRT
<213>  Unknown

<220>
<223>  Synthetic

<400>  2

Tyr Ser Phe Cys Thr Asp His Thr Val Leu Val Gln Thr Arg
1               5                   10

<210>  3
<211>  15
<212>  PRT
<213>  Unknown

<220>
<223>  Synthetic

<400>  3

Val Asp Val Ile Pro Val Asn Leu Pro Gly Glu His Gly Gln Arg
1               5                   10                  15

<210>  4
<211>  14
<212>  PRT
<213>  Unknown

<220>
<223>  Synthetic

<400>  4

Glu Ala Thr Ile Pro Gly His Leu Asn Ser Tyr Thr Ile Lys
1               5                   10


<210>  5
<211>  11
<212>  PRT
<213>  Unknown

<220>
<223>  Synthetic

<400>  5

Ile Tyr Leu Tyr Thr Leu Asn Asp Asn Ala Arg
1               5                   10


<210>  6
<211>  16
<212>  PRT
<213>  Unknown

<220>
<223>  Synthetic

<400>  6

Thr Tyr Leu Gly Asn Ala Leu Val Cys Thr Cys Tyr Gly Gly Ser Arg
1               5                   10                  15


<210>  7
<211>  9
<212>  PRT
<213>  Unknown

<220>
<223>  Synthetic

<400>  7

Ile His Ile Gly Ser Ser Phe Glu Lys
1               5


<210>  8
<211>  24
<212>  PRT
<213>  Unknown

<220>
<223>  Synthetic

<400>  8

Ala Leu Thr Pro Gln Cys Gly Ser Gly Glu Asp Leu Tyr Ile Leu Thr

1               5                   10                  15

Gly Thr Val Pro Ser Asp Tyr Arg
            20

<210>  9
<211>  11
<212>  PRT
<213>  Unknown

<220>
<223>  Synthetic

<400>  9

Val Tyr Gly Ala Leu Met Trp Ser Leu Gly Lys
1               5                   10

<210>  10
<211>  14
<212>  PRT
<213>  Unknown

<220>
<223>  Synthetic

<400>  10

Gly Gly Ser Ala Ser Thr Trp Leu Thr Ala Phe Ala Leu Arg
1               5                   10

<210>  11
<211>  22
<212>  PRT
<213>  Unknown

<220>
<223>  Synthetic

<400>  11

Phe Thr Phe Thr Leu His Leu Glu Thr Pro Lys Pro Ser Ile Ser Ser
1               5                   10                  15

Ser Asn Leu Asn Pro Arg
            20

<210>  12
<211>  19
<212>  PRT
<213>  Unknown

<220>
<223>  Synthetic

<400>  12

Ile Phe Ser Pro Asn Val Val Asn Leu Thr Leu Val Asp Leu Pro Gly
1               5                   10                  15

Met Thr Lys

<210>  13
<211>  9
<212>  PRT
<213>  Unknown

<220>
<223>  Synthetic

<400>  13

Leu Ser Glu Asp Tyr Gly Val Leu Lys
1               5

<210>  14
<211>  17
<212>  PRT
<213>  Unknown

<220>
<223>  Synthetic

<400>  14

Glu Gly Gly Leu Gly Pro Leu Asn Ile Pro Leu Leu Ala Asp Val Thr
1               5                   10                  15

Arg

<210>  15
<211>  15
<212>  PRT
<213>  Unknown

<220>
<223>  Synthetic

<400>  15

Ser Tyr Ser Cys Gln Val Thr His Glu Gly Ser Thr Val Glu Lys
1               5                   10                  15

<210>  16
<211>  15
<212>  PRT
<213>  Unknown

<220>
<223>  Synthetic

<400>  16

Asn Trp Gly Leu Ser Phe Tyr Ala Asp Lys Pro Glu Thr Thr Lys
1               5                   10                  15

<210>  17
<211>  20
<212>  PRT
<213>  Unknown

<220>
<223>  Synthetic

<400>  17

Ala Thr Leu Val Cys Leu Ile Ser Asp Phe Tyr Pro Gly Ala Val Thr
1               5                   10                  15

Val Ala Trp Lys
            20

<210>  18
<211>  23
<212>  PRT
<213>  Unknown

<220>
<223>  Synthetic

<400>  18

Ala Ile Thr Pro Pro His Pro Ala Ser Gln Ala Asn Ile Ile Phe Asp
1               5                   10                  15

Ile Thr Glu Gly Asn Leu Arg
            20

<210>  19
<211>  13
<212>  PRT
<213>  Unknown

<220>
<223>  Synthetic

<400>  19

Val Asn Val Asp Ala Val Gly Gly Glu Ala Leu Gly Arg
1               5                   10

<210>  20
<211>  16
<212>  PRT
<213>  Unknown

<220>
<223>  Synthetic

<400>  20

Glu Ala Ser Met Val Ile Thr Glu Ser Pro Ala Ala Leu Gln Leu Arg
1               5                   10                  15


<210>  21
<211>  15
<212>  PRT
<213>  Unknown

<220>
<223>  Synthetic

<400>  21

His Pro Asp Glu Ala Ala Phe Phe Asp Thr Ala Ser Thr Gly Lys
1               5                   10                  15


<210>  22
<211>  8
<212>  PRT
<213>  Unknown

<220>
<223>  Synthetic

<400>  22

Glu Thr Leu Leu Gln Asp Phe Arg
1               5


<210>  23
<211>  12
<212>  PRT
<213>  Unknown

<220>
<223>  Synthetic

<400>  23

Ala Phe Ile Gln Leu Trp Ala Phe Asp Ala Val Lys
1               5                   10


<210>  24
<211>  13
<212>  PRT
<213>  Unknown

<220>
<223>  Synthetic

<400>  24

Ser Ser Leu Ser Val Pro Tyr Val Ile Val Pro Leu Lys
1               5                   10


<210>  25

<211>  14
<212>  PRT
<213>  Unknown

<220>
<223>  Synthetic

<400>  25

Ile Pro Ile Glu Asp Gly Ser Gly Glu Val Val Leu Ser Arg
1               5                   10


<210>  26
<211>  13
<212>  PRT
<213>  Unknown

<220>
<223>  Synthetic

<400>  26

Ser Leu Leu Glu Gln Tyr His Leu Gly Leu Asp Gln Lys
1               5                   10


<210>  27
<211>  11
<212>  PRT
<213>  Unknown

<220>
<223>  Synthetic

<400>  27

Leu Ser Pro Ile Tyr Asn Leu Val Pro Val Lys
1               5                   10


<210>  28
<211>  10
<212>  PRT
<213>  Unknown

<220>
<223>  Synthetic

<400>  28

Ser Leu His Asp Ala Ile Met Ile Val Arg
1               5                   10


<210>  29
<211>  15
<212>  PRT
<213>  Unknown

<220>
<223>  Synthetic

<400>  29

Asn Ser Ala Thr Gly Glu Glu Ser Ser Thr Ser Leu Thr Val Lys
1               5                   10                  15


<210>  30
<211>  16
<212>  PRT
<213>  Unknown

<220>
<223>  Synthetic

<400>  30

Ser Asp Pro Val Thr Leu Asn Leu Leu His Gly Pro Asp Leu Pro Arg
1               5                   10                  15


<210>  31
<211>  14
<212>  PRT
<213>  Unknown

<220>
<223>  Synthetic

<400>  31

Asp Phe His Ile Asn Leu Phe Gln Val Leu Pro Trp Leu Lys
1               5                   10


<210>  32
<211>  13
<212>  PRT
<213>  Unknown

<220>
<223>  Synthetic

<400>  32

Gly Thr Phe Ala Thr Leu Ser Glu Leu His Cys Asp Lys
1               5                   10


<210>  33
<211>  18
<212>  PRT
<213>  Unknown

<220>
<223>  Synthetic

<400>  33

Leu Tyr His Ser Glu Ala Phe Thr Val Asn Phe Gly Asp Thr Glu Glu
1               5                   10                  15


Ala Lys

<210>  34
<211>  11
<212>  PRT
<213>  Unknown

<220>
<223>  Synthetic

<400>  34

Gly Trp Val Thr Asp Gly Phe Ser Ser Leu Lys
1               5                   10


<210>  35
<211>  15
<212>  PRT
<213>  Unknown

<220>
<223>  Synthetic

<400>  35

Ile His Leu Ile Ser Thr Gln Ser Ala Ile Pro Tyr Ala Leu Arg
1               5                   10                  15


<210>  36
<211>  10
<212>  PRT
<213>  Unknown

<220>
<223>  Synthetic

<400>  36

Asn Gln Val Ser Leu Thr Cys Leu Val Lys
1               5                   10


<210>  37
<211>  12
<212>  PRT
<213>  Unknown

<220>
<223>  Synthetic

<400>  37

Glu Ser Lys Pro Leu Thr Ala Gln Gln Thr Thr Lys
1               5                   10


<210>  38
<211>  16
<212>  PRT
<213>  Unknown

<220>
<223>  Synthetic

<400>  38

Met Val Glu Thr Thr Ala Tyr Ala Leu Leu Thr Ser Leu Asn Leu Lys
1               5                   10                  15


<210>  39
<211>  14
<212>  PRT
<213>  Unknown

<220>
<223>  Synthetic

<400>  39

Leu Pro Lys Pro Tyr Ile Thr Ile Asn Asn Leu Asn Pro Arg
1               5                   10


<210>  40
<211>  13
<212>  PRT
<213>  Unknown

<220>
<223>  Synthetic

<400>  40

Gly Thr Phe Ser Gln Leu Ser Glu Leu His Cys Asp Lys
1               5                   10


<210>  41
<211>  17
<212>  PRT
<213>  Unknown

<220>
<223>  Synthetic

<400>  41

Glu Gln Leu Gly Glu Phe Tyr Glu Ala Leu Asp Cys Leu Cys Ile Pro
1               5                   10                  15


Arg



<210>  42
<211>  12
<212>  PRT
<213>  Unknown

<220>
<223>  Synthetic

<400>  42

Val Ile Ala Ala Glu Gly Glu Met Asn Ala Ser Arg
1               5                   10

<210>  43
<211>  13
<212>  PRT
<213>  Unknown

<220>
<223>  Synthetic

<400>  43

Gly Leu Ile Asp Glu Val Asn Gln Asp Phe Thr Asn Arg
1               5                   10

<210>  44
<211>  10
<212>  PRT
<213>  Unknown

<220>
<223>  Synthetic

<400>  44

Ser Val Ser Ile Gly Tyr Leu Leu Val Lys
1               5                   10

<210>  45
<211>  17
<212>  PRT
<213>  Unknown

<220>
<223>  Synthetic

<400>  45

Val Gln Ala Ala Val Gly Thr Ser Ala Ala Pro Val Pro Ser Asp Asn
1               5                   10                  15

His

<210>  46
<211>  16
<212>  PRT
<213>  Unknown

<220>
<223>  Synthetic

<400>  46

Ser Asn Pro Val Ile Leu Asn Val Leu Tyr Gly Pro Asp Leu Pro Arg
1               5                   10                  15

<210>  47
<211>  13
<212>  PRT
<213>  Unknown

<220>
<223>  Synthetic

<400>  47

Gly Leu Ile Asp Glu Val Asn Gln Asp Phe Thr Asn Arg
1               5                   10

<210>  48
<211>  9
<212>  PRT
<213>  Unknown

<220>
<223>  Synthetic

<400>  48

Val Val Glu Glu Ser Glu Leu Ala Arg
1               5

<210>  49
<211>  13
<212>  PRT
<213>  Unknown

<220>
<223>  Synthetic

<400>  49

Arg Pro Trp Asn Val Ala Ser Leu Ile Tyr Glu Thr Lys
1               5                   10

<210>  50
<211>  13
<212>  PRT
<213>  Unknown

<220>
<223>  Synthetic

<400>  50

Asp Val Phe Leu Gly Met Phe Leu Tyr Glu Tyr Ala Arg
1               5                   10

<210>  51
<211>  22
<212>  PRT

<213>  Unknown

<220>
<223>  Synthetic

<400>  51

Leu Val Arg Pro Glu Val Asp Val Met Cys Thr Ala Phe His Asp Asn
1               5                   10                  15

Glu Glu Thr Phe Leu Lys
            20

<210>  52
<211>  17
<212>  PRT
<213>  Unknown

<220>
<223>  Synthetic

<400>  52

Ala Ala Thr Ile Thr Ala Thr Ser Pro Gly Ala Leu Trp Gly Leu Asp
1               5                   10                  15

Arg

<210>  53
<211>  11
<212>  PRT
<213>  Unknown

<220>
<223>  Synthetic

<400>  53

Ile Thr Leu Pro Asp Phe Thr Gly Asp Leu Arg
1               5                   10

<210>  54
<211>  9
<212>  PRT
<213>  Unknown

<220>
<223>  Synthetic

<400>  54

Thr Asp Phe Leu Ile Phe Asp Pro Lys
1               5

<210>  55
<211>  9

<212>  PRT
<213>  Unknown

<220>
<223>  Synthetic

<400>  55

Leu Phe Ile Pro Gln Ile Thr Thr Lys
1               5


<210>  56
<211>  19
<212>  PRT
<213>  Unknown

<220>
<223>  Synthetic

<400>  56

Asp Ile Pro Gln Pro His Ala Glu Pro Trp Ala Phe Ser Leu Asp Leu
1               5                   10                  15


Gly Leu Lys



<210>  57
<211>  13
<212>  PRT
<213>  Unknown

<220>
<223>  Synthetic

<400>  57

Leu Leu Ser Asp Phe Pro Val Val Pro Thr Ala Thr Arg
1               5                   10


<210>  58
<211>  15
<212>  PRT
<213>  Unknown

<220>
<223>  Synthetic

<400>  58

Ile Glu Ile Asn Phe Pro Ala Glu Tyr Pro Phe Lys Pro Pro Lys
1               5                   10                  15


<210>  59
<211>  12
<212>  PRT
<213>  Unknown

<220>
<223>  Synthetic

<400>  59

Phe Pro Val Glu Met Thr His Asn His Asn Phe Arg
1               5                   10